# EUROPEAN PATENT APPLICATION

(11) **EP 4 253 359 A1**
(43) Date of publication of application: **04.10.2023**
(21) Application number: 21897403.8
(22) Date of filing: 21.07.2021
(51) Int. Cl.: C07C 67/055, B01J 8/06, B01J 23/89, C07C 69/155, C07B 61/00

(54) **FIXED BED MULTI-TUBULAR REACTOR FOR PRODUCING ALKENYL ACETATE**

(30) Priority: 27.11.2020 JP 2020197024
(71) Applicant: Resonac Corporation, Tokyo 105-8518 (JP)
(72) Inventor: IWAMA, Yasuhiro, Oita-shi, Oita 870-0189 (JP); IKUSHIMA, Maiko, Oita-shi, Oita 870-0189 (JP); UMEHARA, Kazuki, Oita-shi, Oita 870-0189 (JP); MIURA, Shuhei, Oita-shi, Oita 870-0189 (JP); MAKI, Toshiyuki, Oita-shi, Oita 870-0189 (JP); ETO, Kensho, Oita-shi, Oita 870-0189 (JP)
(74) Representative: Strehl Schübel-Hopf & Partner
(86) International application number: PCT/JP2021/027393
(87) International publication number: WO 2022/113423

(57) **Abstract**

Provided is a reactor device in which a fixed bed multi-tubular reactor is used to produce alkenyl acetate through a gas phase catalytic oxidation reaction of a lower olefin, acetic acid, and oxygen, and which enables accurate measurement of the temperature of a catalyst layer in a reaction tube even when a process operation has been carried out for a long time. A fixed bed multi-tubular reactor for producing alkenyl acetate, wherein the fixed bed multi-tubular reactor is equipped with a plurality of reaction tubes to which a raw material gas and a mist of an aqueous solution of an alkali metal acetate are supplied from the upper portion of the fixed bed multi-tubular reactor, a thermometer protection tube inserted into at least one of the plurality of reaction tubes from the lower portion of the fixed bed multi-tubular reactor, and a thermometer inserted into the thermometer protection tube.

## Description

### FIELD

The present invention relates to a fixed-bed multi-tubular reactor which is used in the production of alkenyl acetate such as allyl acetate and vinyl acetate from a lower olefin, acetic acid, and oxygen by a gas-phase catalytic oxidation reaction.

### BACKGROUND

Allyl acetate is one of the important industrial materials used as raw materials for the production of solvents and allyl alcohol.

As a method for producing allyl acetate, there is a method using a gas phase reaction or a liquid phase reaction of propylene, acetic acid, and oxygen as raw materials. As the catalyst used for this reaction, a catalyst comprising palladium as a main catalyst component and an alkali metal or alkaline earth metal compound as a co-catalyst supported on a carrier is known and widely used. For example, JP H02-91045 A (Patent Literature 1) describes a method for producing allyl acetate using a catalyst in which palladium, potassium acetate, and copper are supported on a carrier.

Vinyl acetate is an important industrial material that is used as a raw material for vinyl acetate resins, a raw material for polyvinyl alcohol, and a monomer for copolymerization with ethylene, styrene, an acrylate, or a methacrylate in a wide range of fields such as paints, adhesives, and fiber treatment agents.

As a method for producing vinyl acetate, there is a method using a gas phase reaction or a liquid phase reaction of ethylene, acetic acid, and oxygen as raw materials. As the catalyst used for this reaction, a catalyst comprising palladium as a main catalyst component and an alkali metal or alkaline earth metal compound as a co-catalyst supported on a carrier is known and widely used. For example, JP 2004-526553 A (Patent Literature 2) describes a method for producing vinyl acetate using a catalyst in which palladium, gold, and potassium acetate are supported on a carrier.

Patent Literature 1 and the Series "Catalysts and Economy", Vol. 35, No. 7 (1993), pp. 467-470 (Non-Patent Literature 1) describe that in the alkenyl acetate production process using the catalyst described above, since potassium acetate gradually leaks out from the catalyst filled in the reaction tube during long-term continuous reaction process operation over thousands of hours, it is necessary to continuously supply potassium acetate to the catalyst.

Fixed-bed tubular reactors are generally used as the reactor for the production of alkenyl acetate. A fixed-bed tubular reactor is a reaction tube filled with a catalyst (supported on a carrier) as a fixed-bed. A fixed-bed multi-tubular reactor is a fixed-bed tubular reactor having a plurality of reaction tubes. A reaction substrate is supplied to the reaction tubes in a gaseous state, reacts in the catalyst layer, and the reaction product is discharged from the reaction tube. As the reaction tube, a straight reaction tube is often used from the viewpoints of equipment production, equipment maintenance, workability during catalyst filling and replacement, and the removal of reaction heat. The reaction tube is often installed in the vertical direction (vertical-type) from the viewpoint of workability of filling and unloading the catalyst.

The catalyst layer temperature of such a reactor is generally monitored to confirm the reaction state of the catalyst layer during operation of the industrial production process. As a method for measuring the temperature of the catalyst layer, for example, as described in JP 2002-212127 A (Patent Literature 3), there is a method wherein before filling the catalyst, protection tubes (sheaths) are installed in several reaction tubes representing the fixed-bed multi-tubular reactor as a whole, thermocouples are inserted into these protection tubes, and the temperatures are measured in the longitudinal direction inside the reaction tubes.

Since the gas-phase catalytic oxidation reaction is an exothermic reaction, a heat medium is generally supplied to the outside of the reaction tube for heat removal. By monitoring the temperature difference between the temperature of the heat medium (shell temperature) outside the reaction tube and the temperature of the catalyst layer, it is possible to observe the level of the reaction of the reaction substrate at a certain position in the vertical direction of the catalyst layer. When the temperature distribution is unevenly distributed, by controlling the reaction based on the temperature unevenness, the plant can be operated so that the gas-phase catalytic oxidation reaction proceeds stably and highly efficiently.

### [CITATION LIST]

### [PATENT LITERATURE]

[PTL 1] JP H02-91045 A
[PTL 2] JP 2004-526553 A
[PTL 3] JP 2002-212127 A

### [NON-PATENT LITERATURE]

[NPL 1] Series "Catalysts and Economy", "Transition of Vinyl Acetate Manufacturing Process and Its Prospect", Vol. 35, No. 7 (1993), pp. 467-470

### SUMMARY

### [TECHNICAL PROBLEM]

A thermocouple inserted inside a reaction tube of a fixed-bed tubular reactor as described above can accurately measure the temperature of the catalyst layer when only gas is circulated as the process fluid. However, the present inventors have discovered that when this temperature measurement method is applied to a fixed-bed multi-tubular reactor for alkenyl acetate production and a long-term continuous reaction is carried out for several months, though the reaction proceeds and the desired reaction product is produced in the fixed-bed multi-tubular reactor as a whole (this means that the temperature of the catalyst layer was higher than the heat medium temperature (shell temperature) of the shell side (heat medium distribution area outside the reaction tube) during the continuous reaction due to the generation of reaction heat), in reality, the temperature difference between the catalyst layer temperature and the shell temperature is no longer observed, whereby a catalyst layer temperature representing the fixed-bed multi-tubular reactor as a whole cannot be monitored.

In light of the circumstances described above, an object of the present invention is to provide a reaction apparatus for the production of alkenyl acetate by gas-phase catalytic oxidation reaction of a lower olefin, acetic acid, and oxygen using a fixed-bed multi-tubular reactor, in which the temperature of a catalyst layer inside a reaction tube can accurately be measured even during long-term process operation.

### [SOLUTION TO PROBLEM]

The present inventors have established that the above phenomenon is caused when a mist of an aqueous solution of an alkaline metal acetate supplied to a fixed-bed multi-tubular reactor for vinyl acetate production and allyl acetate production attaches to the thermometer protection tube inserted into the reactor, then becomes droplets and the droplets flow down along the thermometer protection tube, and the alkaline metal acetate is selectively supplied to the reaction tube having the thermometer protection tube inserted therein, whereby the amount of alkali metal acetate supported by the catalyst in the reaction tube having the thermometer protection tube inserted therein becomes excessive, resulting in a decrease in catalytic activity. Since the amount of reaction heat generated is small in a reaction tube filled with a catalyst having reduced catalytic activity, i.e., a reaction tube having a thermocouple inserted therein, the temperature difference between the catalyst layer temperature and the shell temperature in these reaction tubes becomes small. Conversely, in the reaction tubes having no thermocouple inserted therein, the reaction proceeds appropriately, whereby the catalyst layer temperatures in these reaction tubes are correspondingly higher than the shell temperatures, but this is not reflected in plant catalyst layer temperature readings. Thus, it is sometimes impossible to properly operate the plant.

In light of the foregoing, the present inventors have discovered that in order to prevent droplets of alkali metal acetate attached to the thermometer protection tube from being supplied along the thermometer protection tube to the reaction tube having the thermometer protection tube inserted therein, by inserting the thermometer protection tube from the reactor bottom part, the selective supply of droplets of the alkali metal acetate to the reaction tube having the thermometer protection tube inserted therein can be prevented, and have completed the present invention.

Specifically, the present invention encompasses the following [1] to [4].
[1] A fixed-bed multi-tubular reactor for alkenyl acetate production, comprising:
   a plurality of reaction tubes which are supplied with a raw material gas and a mist of an aqueous solution of an alkali metal acetate from a top part of the fixed-bed multi-tubular reactor,
   a thermometer protection tube which is inserted into at least one of the plurality of reaction tubes from a bottom part of the fixed-bed multi-tubular reactor, and
   a thermometer which is inserted into the thermometer protection tube.
[2] The fixed-bed multi-tubular reactor according to [1], wherein the alkali metal acetate is at least one selected from the group consisting of potassium acetate and cesium acetate.
[3] The fixed-bed multi-tubular reactor according to [1] or [2], wherein a number of the reaction tubes having the thermometer protection tube inserted therein is 3 to 10.
[4] The fixed-bed multi-tubular reactor according to any one of [1] to [3], wherein the thermometer is a thermocouple or a resistance thermometer.

### [ADVANTAGEOUS EFFECTS OF INVENTION]

According to the present invention, since the temperature of a catalyst layer inside a reaction tube during alkenyl acetate production can be accurately measured at all times, the measured catalyst layer temperature can be used for the detection of a hot spot or as an index for adjusting the supply of alkali metal acetate. This makes it possible to maintain a high production efficiency of alkenyl acetate over long periods of time.

### BRIEF DESCRIPTION OF DRAWINGS

FIG. 1 is a schematic longitudinal cross-sectional view of a fixed-bed multi-tubular reactor according to an embodiment.
FIG. 1A is a bottom view of the fixed-bed multi-tubular reactor of FIG. 1 taken along plane A-A'.
FIG. 2A is a schematic view showing a catalyst filling state of a reaction tube of Example 1 before and after reaction.
FIG. 2B is a schematic view showing a catalyst filling state of a reaction tube of Comparative Example 1 before and after reaction.
FIG. 2C is a schematic view showing a catalyst filling state of a reaction tube of Reference Example 1 before and after reaction.
FIG. 3A is a graph showing the relationship of the temperature difference between the catalyst layer temperature and the shell temperature with the total operating time of Example 1.
FIG. 3B is a graph showing the relationship of the temperature difference between the catalyst layer temperature and the shell temperature with the total operating time of Comparative Example 1.

### DESCRIPTION OF EMBODIMENTS

The preferred embodiments of the present invention will be described below, but the present invention is not limited to only these embodiments.

### <Fixed-bed Multi-tubular Reactor>

The fixed-bed multi-tubular reactor for alkenyl acetate production according to an embodiment comprises a plurality of reaction tubes which are supplied with a raw material gas and a mist of an aqueous solution of an alkali metal acetate from a top part of the fixed-bed multi-tubular reactor, a thermometer protection tube which is inserted into at least one of the plurality of reaction tubes from a bottom part of the fixed-bed multi-tubular reactor, and a thermometer which is inserted into the thermometer protection tube.

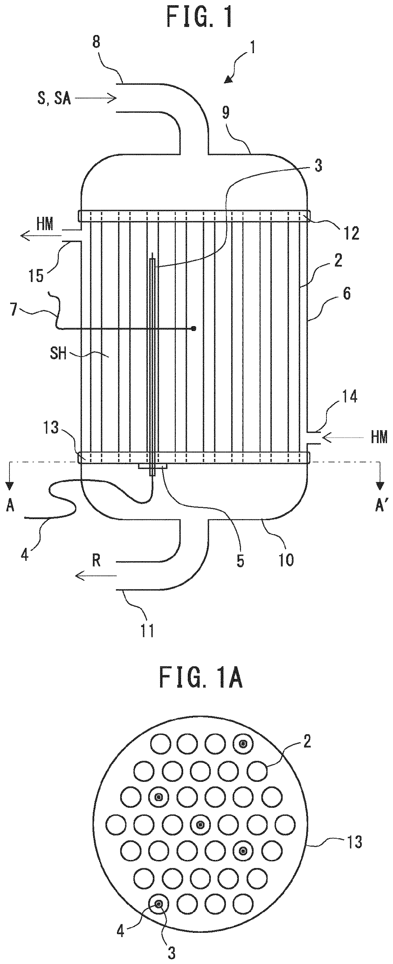

FIG. 1 is a schematic longitudinal cross-sectional view of a fixed-bed multi-tubular reactor (hereinafter also referred simply to as a "reactor") according to an embodiment, and FIG. 1A is a bottom view of the reactor 1 of FIG. 1 taken along a plane A-A'. As shown in FIG. 1, the reactor 1 comprises a plurality of reaction tubes 2, a thermometer protection tube 3 which is inserted into at least one of the plurality of reaction tubes 2 from the bottom part of the reactor 1, and a thermometer 4 which is inserted into the thermometer protection tube 3. The thermometer protection tube 3 may be affixed by a holding device 5 to prevent it from falling downwards. The holding device 5 preferably has as large an opening as possible so as to minimize obstruction of the flow of reaction gases.

The interior of the reaction tube 2 is filled with a catalyst (supported on a carrier, not illustrated) as a fixed-bed. A reaction substrate is supplied as a raw material gas S in a gaseous state through supply piping 8 to the reaction tube 2 from a raw material gas supply part 9 located at the top part of the reactor 1, and reacts in the catalyst layer to produce a reaction product R. After exiting the reaction tube 2, the reaction product R is collected in a reaction product discharge part 10 at the bottom part of the reactor 1 and discharged through extraction piping 11. The reaction tube 2 is preferably a straight pipe from the viewpoints of equipment production, equipment maintenance, workability during catalyst filling and replacement, and removal of reaction heat. The reaction tube 2 is preferably installed in the vertical direction (vertical type) from the viewpoint of workability of filling and unloading the catalyst. The upper end and the lower end of the reaction tube 2 are affixed by an upper affixation plate 12 and a lower affixation plate 13, respectively.

Since the gas-phase catalytic oxidation reaction for synthesizing the alkenyl acetate is an exothermic reaction, a system for removing reaction heat from the outside of the reaction tube 2 is required. The inner diameter, outer diameter, length, material, reaction heat removal device, and reaction heat removal method of the reaction tube 2 are not particularly limited, and from the viewpoints of reaction heat removal efficiency, and balance between heat exchange area and pressure loss inside the reaction tube, the reaction tube 2 preferably has an inner diameter of 10 to 40 mm and a length of 1 to 8 m. Since there is a limit to increasing the inner diameter of the reaction tube 2 in order to remove reaction heat, the reactor 1 is configured as a multi-tubular reactor having a plurality of reaction tubes 2. The number of reaction tubes 2 is preferably, for example, 1000 to 20000 from the viewpoint of ensuring production volume. The material of the reaction tube is preferably SUS because it has excellent heat resistance and corrosion resistance.

The reactor 1 has a jacket 6 in the shape of a cylinder or square tube for cooling the reaction tube 2 (heating at the start of the reaction). A heat medium inlet 14 is provided above the lower affixation plate 13 on the side of the jacket 6, and a heat medium outlet 15 is provided below the upper affixation plate 12 on the side of the jacket 6, respectively. The space defined by the jacket 6, the upper affixation plate 12, the lower affixation plate 13, and the outside of the reaction tube 2 is referred to as a shell SH. A heat medium HM for controlling the temperature of the reaction tube 2 is introduced from the heat medium inlet 14, flows through the shell SH, and is discharged from the heat medium outlet 15. One or more baffles may be provided inside the shell SH for regulating the flow direction of the heat medium HM and making the temperature distribution of the heat medium HM more uniform throughout the shell SH. The temperature of heat medium HM flowing through the shell SH is measured by a shell thermometer 7. The shell thermometer 7 is preferably arranged so that the temperature measurement part thereof is positioned at the center of the reactor 1 (in the case of a cylindrical reactor, the center of the cross-sectional circle and the middle point of the height of the cylinder). The heat medium HM is preferably water (steam).

A raw material gas S and a mist of an aqueous solution of alkali metal acetate SA are supplied to the reaction tube 2 through the supply piping 8.

The raw material gas S is a lower olefin such as ethylene or propylene, acetic acid, and oxygen gas. The lower olefin is preferable ethylene or propylene.

The mist of the aqueous solution of alkali metal acetate SA can be formed by spraying an aqueous solution of alkali metal acetate SA into the raw material gas S. The alkali metal acetate SA is preferably at least one selected from the group consisting of potassium acetate and cesium acetate. The concentration of the aqueous solution of alkali metal acetate SA is preferably 0.1 to 20 mass%. The concentration of the aqueous solution of alkali metal acetate SA may be increased or decreased depending on the total reaction time. The supply rate of alkali metal acetate SA is preferably 2 to 200 mg/h per liter volume of the catalyst layer.

In addition to the raw material gas S and the mist of the aqueous solution of alkali metal acetate SA, the reaction tube 2 may be supplied with water, an inert gas, or both through the supply piping 8. The inert gas is preferably nitrogen gas, carbon dioxide, or a mixed gas thereof.

The reaction product R, unreacted gas, etc., are extracted through the extraction piping 11. The reaction product R is vinyl acetate when the raw material gas S is ethylene and allyl acetate when it is propylene.

The thermometer protection tube 3 is inserted into at least one of the reaction tubes 2 from the bottom part of the reactor 1. The thermometer protection tube 3 is preferably inserted up to near the top part of the reaction tube 2. The number of reaction tubes 2 having the thermometer protection tube 3 inserted therein is preferably 3 to 10. When there are a plurality of thermometer protection tubes 3, these thermometer protection tubes 3 are preferably evenly or symmetrically arranged inside the reactor 1. When there is one thermometer protection tube 3, it is preferable that the thermometer protection tube 3 be arranged in the center of the reactor 1. In FIG. 1A, thermometer protection tubes 3 are inserted into five reaction tubes 2 including a central reaction tube 2, and a thermometer 4 is inserted into each of the thermometer protection tubes 3.

The diameter of the thermometer protection tube 3 is preferably 1/6 to 1/2 of the inner diameter of the reaction tube 2, and more preferably 1/4 to 1/2 of the inner diameter of the reaction tube 2. When the thermometer protection tube 3 is excessively thick, the amount of catalyst filled in the reaction tube 2 decreases, the cross-sectional area through which the raw material gas S flows decreases, and pressure loss thus increases, whereby the reaction amount in the reaction tube 2 having the thermometer protection tube 3 inserted therein relatively decreases, i.e., a temperature rise due to the reaction heat of the entire reactor 1 sometimes deviates from the measured value of the thermometer 4. The material of the thermometer protection tube 3 is preferably SUS because of its excellent heat resistance and corrosion resistance.

The thermometer 4 is inserted into the thermometer protection tube 3. The thermometer is preferably a thermocouple or a resistance thermometer. When a thermocouple capable of multipoint measurement is used, the catalyst layer temperatures can be measured at a plurality of positions (heights) of the reaction tube 2.

### <Catalyst for Alkenyl Acetate Production>

The catalyst for alkenyl acetate production filled in the reaction tube 2 is not particularly limited as long as it is a solid catalyst, and conventionally known catalysts can be used depending on the reaction. For example, a catalyst comprising palladium as a main catalyst component and an alkali metal or alkaline earth metal compound as a co-catalyst supported on a carrier, as described in JP H02-91045 A (Patent Literature 1) described above, can be used.

The method for preparing such a catalyst is also not particularly limited, and various conventionally well-known methods can be used. The raw materials used for preparing the catalyst are not particularly limited, and nitrates, carbonates, acetates, ammonium salts, oxides, or halides of each element can be used in combination.

The catalyst for producing allyl acetate used in one embodiment comprises (a) palladium, (b) gold, (c) a compound containing at least one element selected from the group consisting of copper, nickel, zinc, and cobalt, (d) an alkali metal acetate, and (e) a carrier.

The vinyl acetate production catalyst used in one embodiment comprises (a) palladium, (b) gold, (d) an alkali metal acetate, and (e) a carrier. These components are described below.

### (a) Palladium

(a) Palladium may have any valence, but is preferably metallic palladium. "Metallic palladium" as used herein refers to palladium having a valence of zero. Metallic palladium can usually be obtained by reducing divalent or tetravalent palladium ions using a reducing agent such as hydrazine or hydrogen. In this case, the palladium may not be entirely in the metallic state.

The raw material of palladium or the compound containing palladium is not particularly limited, and metallic palladium or a palladium precursor which can be converted to metallic palladium can be used. In the present disclosure, metallic palladium and palladium precursors are collectively referred to as "palladium sources." Examples of palladium precursors include palladium chloride, palladium nitrate, palladium sulfate, palladium sodium chloride, palladium potassium chloride, palladium barium chloride, and palladium acetate. Palladium sodium chloride is preferably used. As the palladium precursor, a single compound may be used, or a plurality of compounds may be used in combination.

The mass ratio of (a) palladium and (e) carrier in the catalyst is preferably (a):(e) = 1:10 to 1:1000, and more preferably (a):(e) = 1:20 to 1:500. This ratio is defined as the ratio of the mass of elemental palladium to the mass of the carrier.

### (b) Gold

(b) Gold is supported on the carrier in the form of a compound containing elemental gold, but it is preferable that substantially the entirety thereof be ultimately metallic gold. "Metallic gold" as used herein refers to gold having a valence of zero. Metallic gold can usually be obtained by reducing monovalent or trivalent gold ions using a reducing agent such as hydrazine or hydrogen gas. In this case, the gold may not be entirely in the metallic state.

The raw material of gold or the compound containing gold is not particularly limited, and metallic gold or a gold precursor which can be converted to metallic gold can be used. In the present disclosure, metallic gold and gold precursors are collectively referred to as "gold sources." Examples of gold precursors include chloroauric acid, sodium chloroaurate, and potassium chloroaurate. Chloroauric acid or sodium chloroaurate is preferably used. As the gold precursor, a single compound may be used, or a plurality of compounds may be used in combination.

The mass ratio of (b) gold and (e) carrier in the catalyst is preferably (b):(e) = 1:40 to 1:65000, more preferably (b):(e) = 1:70 to 1:16000, and further preferably (b):(e) = 1:100 to 1:5000. This ratio is defined as the ratio of the mass of elemental gold to the mass of the carrier.

### (c) Compound Containing at Least One Element Selected from Group Consisting of Copper, Nickel, Zinc, and Cobalt (also simply referred to as "(c) forth periodic metal compounds" in the present disclosure)

As the (c) forth periodic metal compound, soluble salts such as nitrates, carbonates, sulfates, organic acid salts and halides of at least one element selected from the group consisting of copper, nickel, zinc, and cobalt can be used. Examples of organic acid salts include acetate. In general, compounds which are readily available and water soluble are preferable. Examples of preferable compounds include copper nitrate, copper acetate, nickel nitrate, nickel acetate, zinc nitrate, zinc acetate, cobalt nitrate, and cobalt acetate. Among these, copper acetate is most preferable from the viewpoint of stability of the raw material and availability. As the (c) forth periodic metal compound, a single compound may be used, or a plurality of compounds may be used in combination.

The mass ratio of (c) forth periodic metal compound and (e) carrier in the catalyst for producing allyl acetate is preferably (c):(e) = 1: 10 to 1:500, and more preferably (c):(e) = 1:20 to 1 :400. This ratio is defined as the ratio of the total mass of elemental copper, nickel, zinc, or cobalt to the mass of the carrier.

### (d) Alkali Metal Acetate

The (d) alkali metal acetate is preferably an acetate of at least one alkali metal selected from the group consisting of lithium, sodium, potassium, rubidium, and cesium. Specifically, potassium acetate, sodium acetate, and cesium acetate are preferable, and potassium acetate and cesium acetate are more preferable.

The mass ratio of (d) alkali metal acetate and (e) carrier in the catalyst is preferably (d):(e) = 1:2 to 1:50, and more preferably (d):(e) = 1:3 to 1:40. This ratio is defined as the ratio of the mass of the alkali metal acetate used and the mass of the carrier.

### (e) Carrier

The (e) carrier is not particularly limited, and porous substances which are generally used as carriers for catalysts can be used. Examples of preferable carriers include silica, alumina, silica-alumina, diatomaceous earth, montmorillonite, titania, and zirconia, with silica being more preferable. When a carrier containing silica as a main component is used, the silica content of the carrier is preferably at least 50 mass%, more preferably at least 90 weight%, relative to the mass of the carrier.

The carrier preferably has a specific surface area of 10 to 1000 m²/g, more preferably 100 to 500 m²/g, as measured by the BET method. The bulk density of the carrier is preferably in the range of 50 to 1000 g/L, and more preferably in the range of 300 to 500 g/L. The water absorption rate of the carrier is preferably 0.05 to 3 g-water/g-carrier, and more preferably 0.1 to 2 g-water/g-carrier. Regarding the pore structure of the carrier, the average pore diameter thereof is preferably in the range of 1 to 1000 nm, and more preferably in the range of 2 to 800 nm. When the average pore diameter is 1 nm or more, diffusion of gas can be facilitated. Conversely, when the average pore diameter is 1000 nm or less, the specific surface area of the carrier necessary for obtaining catalytic activity can be secured.

The shape of the carrier is not particularly limited. Specific examples thereof include powder-like, spherical, and pellet-like shapes, but the shape is not limited to these. The optimal shape can be selected in accordance with the reaction type, reactor, etc., used.

The particle size of the carrier is also not particularly limited. When the carrier is spherical, the particle diameter thereof is preferably in the range of 1 to 10 mm, and more preferably in the range of 2 to 8 mm. When the reaction tube 2 is filled with the catalyst and the reaction is carried out, if the particle diameter is 1 mm or more, an excessive increase in pressure loss when the gas is circulated can be prevented, whereby the gas can be effectively circulated. Conversely, if the particle diameter is 10 mm or less, the raw material gas can easily be diffused into the interior of the catalyst, whereby the catalytic reaction can proceed effectively.

### <Filling Reaction Tube 2 with Catalyst for Alkenyl Acetate Production>

The reaction tube 2 of the reactor 1 may be uniformly filled with the catalyst for alkenyl acetate production or two or more catalyst layers containing the catalyst for alkenyl acetate production with different amounts of alkali metal salt may be arranged such that the amount of alkali metal acetate supported on the carrier decreases sequentially from the inlet side to the outlet side of the reactor 1 along the flow direction (reaction direction) of the raw material gas.

### <Production of Alkenyl Acetate>

The reaction to produce alkenyl acetate is carried out in the gas phase using a lower olefin, acetic acid, and oxygen as raw materials. For example, when the lower olefin is ethylene, the reaction formula is as shown in formula (1), and when it is propylene, the reaction formula is as shown in formula (2).

CH₂=CH₂ + CH₃COOH + 1/2O₂ → CH₂=CHOCOCH₃ + H₂O (1)

CH₂=CHCH₃ + CH₃COOH + 1/2O₂ → CH₂=CHCH₂OCOCH₃ + H₂O (2)

The molar ratio of acetic acid, the lower olefin, and oxygen in the raw material gas is preferably acetic acid:lower olefin:oxygen = 1:0.08 to 16:0.01 to 4. When the lower olefin is ethylene, it is preferable that acetic acid:ethylene:oxygen = 1:0.2 to 9:0.07 to 2. When the lower olefin is propylene, it is preferable that acetic acid:propylene:oxygen = 1: 1 to 12:0.5 to 2.

The raw material gas contains a lower olefin, acetic acid, and oxygen gas, and may further contain nitrogen gas, carbon dioxide, or a noble gas as a diluent, if necessary. When the lower olefin, acetic acid, and oxygen gas are defined as the reaction raw materials, the ratio of the reaction raw materials and the diluent is preferably reaction raw material:diluent = 1:0.05 to 9, and more preferably reaction raw material: diluent = 1:0.1 to 3, as a molar ratio.

The raw material gas preferably contains 0.5 to 25 mol% of water, and more preferably 1 to 20 mol% thereof. Without bound by theory, it is believed that the presence of water in the reaction system reduces efflux of the (d) alkali metal acetate from the catalyst. Even if a large amount of water exceeding 25 mol% is present, the above effect is not improved, and there is a risk that hydrolysis of the produced alkenyl acetate will proceed.

The raw material gas is preferably supplied to the reactor 1 at a space velocity of 10 to 15000 h⁻¹, and more preferably 300 to 8000 h⁻¹ under standard conditions. By setting the space velocity to 10 h⁻¹ or more, the heat of reaction can be appropriately removed. Conversely, by setting the space velocity to 15000 h⁻¹ or less, equipment such as the compressor can be of a practical size.

The reaction temperature is preferably in the range of 100 to 300°C, and more preferably in the range of 120 to 250°C. By setting the reaction temperature to 100°C or higher, the reaction rate can be set within a practical range. By setting the reaction temperature to 300°C or lower, the heat of reaction can be appropriately removed.

The reaction pressure is preferably in the range of 0 to 3 MPaG (gauge pressure), and more preferably in the range of 0.1 to 1.5 MPaG. By setting the reaction pressure to 0 MPaG or higher, the reaction rate can be set within a practical range. By setting the reaction pressure to 3 MPaG or less, an increase in cost related to equipment such as the reaction tube can be suppressed.

The lower olefin such as ethylene and propylene contained in the raw material gas is not particularly limited. Generally, it is preferable to use a high-purity olefin, but the olefin may be mixed with a lower saturated hydrocarbon such as methane, ethane, or propane.

The oxygen gas is not particularly limited. The oxygen gas diluted with an inert gas such as nitrogen gas or carbon dioxide gas, for example, in the form of air can be supplied, but when the gas after the reaction is circulated, it is generally advantageous to use oxygen of a high concentration, preferably oxygen having a purity of 99% by volume or more.

### EXAMPLES

The present invention will be further described below with reference to the Examples and Comparative Examples, but the present invention is not limited to these Examples.

### <Preparation Example 1 - Preparation of Catalyst A>

Catalyst A was prepared by the following procedure using a spherical silica carrier (sphere diameter: 5 mm, specific surface area: 155 m²/g, water absorption rate: 0.85 g-water/g-carrier, hereinafter simply referred to as "silica carrier").

### Step 1

4.1 L of an aqueous solution containing 199 g of palladium sodium chloride and 4.08 g of sodium chloroaurate tetrahydrate was prepared and used as solution A-1. 12 L of the silica carrier (bulk density: 473 g/L, water absorption amount: 402 g/L) was added thereto and impregnated with the A-1 solution to absorb the entire amount thereof.

### Step 2

427 g of sodium metasilicate nonahydrate was dissolved in pure water, and diluted with pure water using a graduated cylinder so that the total amount thereof was 8.64 L to obtain solution A-2. The metal-supported carrier (A-1) obtained in step 1 was impregnated with the solution A-2 and allowed to stand at room temperature (23°C) for 20 hours.

### Step 3

300 g of hydrazine monohydrate was added to the slurry of the alkali-treated silica carrier (A-2) obtained in step 2, gently stirred, and allowed to stand at room temperature for 4 hours. After filtering the obtained catalyst, it was transferred to a glass column equipped with a stopcock, and pure water was passed therethrough for 40 hours for washing. This was then dried at 110°C for 4 hours under an air stream to obtain a metal-supported catalyst (A-3).

### Step 4

624 g of potassium acetate and 90 g of copper acetate monohydrate were dissolved in pure water and diluted with pure water using a graduated cylinder so that the total amount was 3.89 L. The metal-supported catalyst (A-3) obtained in step 3 was added thereto to absorb the entire amount thereof. This was then dried at 110°C for 20 hours under an air stream to obtain catalyst A for allyl acetate production.

The mass ratio of (a) palladium, (b) gold, (c) forth periodic metal compound (copper), and (d) alkali metal acetate (potassium acetate) was (a):(b):(c):(d) = 1:0.024:0.39:8.5. This mass ratio is based on the masses of the constituent elements for (a), (b) and (c) and the mass of the alkali metal acetate for (d). The loading amount (g) of the (d) alkali metal acetate per g of the (e) carrier was 0.110 g/g.

The amount of potassium acetate, which was the alkali metal acetate in the catalyst, was quantified as the content (mass%) of the K (potassium) atoms using the absolute calibration curve method by X-ray fluorescence analysis (XRF) after the catalyst was pulverized into a uniform powder and then molded.

### <Preparation Example 2 - Preparation of Catalyst B>

Catalyst B was produced by repeating the operation of Preparation Example 1 except that in step 4, the amount of potassium acetate was changed from 624 g to 396 g. The mass ratio of (a), (b), (c), and (d) was (a):(b):(c):(d) = 1:0.024:0.39:5.4. The loading amount (g) of the (d) alkali metal acetate (potassium acetate) per g of the (e) carrier was 0.069 g/g.

### Example 1

Allyl acetate was produced using a fixed-bed multi-tubular reactor 1 as shown in FIG. 1. The number of reaction tubes 2 was approximately 5000, and the reaction tubes 2 were arranged in a hexagonal lattice. Each reaction tube 2 had a length of approximately 6.3 m and an inner diameter of 34 mm. The reaction tube 2 was filled, in order from the raw material gas inlet side (upper) to the outlet side, so as to achieve a layer length of 0.8 m of inert balls placed on the upstream side of the catalyst on the raw material gas inlet side, a layer length of 3.3 m of the catalyst A having a high potassium acetate loading amount and a high activity, and a layer length of 2.2 m of the catalyst B having a low potassium acetate loading amount and a low activity.

Thermometer protection tubes 3 having an outer diameter of 8 mm and an inner diameter of 6 mm were inserted into three of the reaction tubes 2 from the bottom part of the reactor 1. In each thermometer protection tube 3, a multipoint thermocouple capable of measuring temperatures at different heights (top part, middle part, and bottom part of the catalyst layer) was inserted as the thermometer 4 to monitor the temperatures of the catalyst layer during the reaction. The shell temperature was measured by a thermocouple arranged as a shell thermometer 7 in the center of reactor 1.

A raw material gas having the composition shown in Table 1 was circulated at a space velocity of 2000 h⁻¹, and the reaction was carried out under the conditions of a reaction temperature of 160°C and a reaction pressure of 0.75 MPaG (gauge pressure). An aqueous solution of potassium acetate (1.5 mass%) was sprayed into the raw material gas from a spray nozzle at a supply rate of 24 g/h.

### [Table 1]

**Table 1**

| Component | Content (volume%) |
|---|---|
| Acetic acid (vaporized) | 8 |
| Propylene | 35 |
| Oxygen gas | 6 |
| Water | 18 |
| Nitrogen gas | 33 |

The reaction continued for 7000 hours to continuously produce allyl acetate.

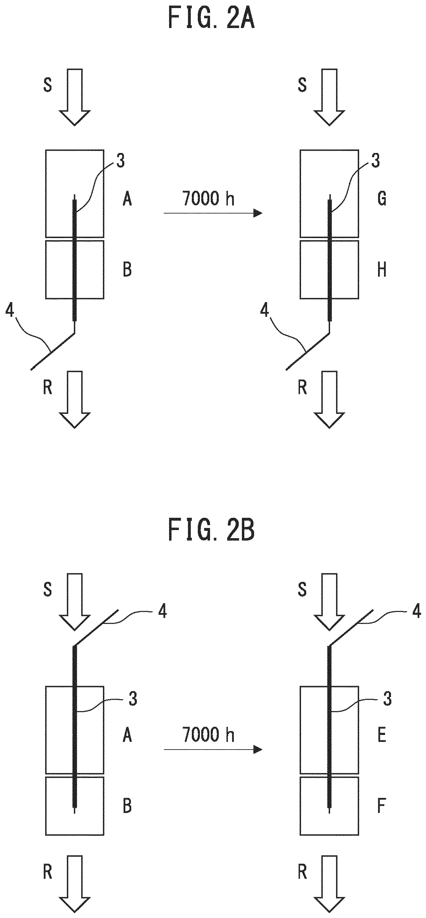

After completion of the reaction, the catalyst was separated at a ratio of 3:2 from the inlet side of the raw material gas and extracted from a reaction tube 2 having a thermometer protection tube 3 inserted therein from the bottom part of the reactor 1, and the catalyst on the inlet side of the reaction tube 2 was designated as catalyst G and the catalyst on the outlet side of the reaction tube 2 was designated as catalyst H. FIG. 2A schematically shows the catalyst filling state of the reaction tube of Example 1 before and after reaction.

### Comparative Example 1

Allyl acetate was produced in the same manner as in Example 1, except that the thermometer protection tube 3 was inserted into the reaction tube 2 from the top part of the reactor 1.

After completion of the reaction, the catalyst was separated at a ratio of 3:2 from the inlet side of the raw material gas and extracted from a reaction tube 2 having a thermometer protection tube 3 inserted therein from the bottom part of the reactor 1, and the catalyst on the inlet side of the reaction tube 2 was designated as catalyst E and the catalyst on the outlet side of the reaction tube 2 was designated as catalyst F. FIG. 2B schematically shows the catalyst filling state of the reaction tube of Comparative Example 1 before and after reaction.

### Reference Example 1

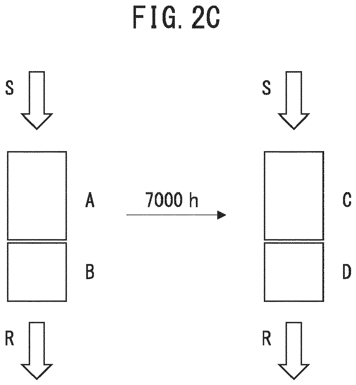

In Example 1, after completion of the reaction, the catalyst was separated at a ratio of 3:2 from the inlet side of the raw material gas and extracted from a reaction tube 2 having no thermometer protection tube 3 inserted therein, and the catalyst on the inlet side of the reaction tube 2 was designated as catalyst C and the catalyst on the outlet side of the reaction tube 2 was designated as catalyst D. FIG. 2C schematically shows the catalyst filling state of the reaction tube of Reference Example 1 before and after reaction.

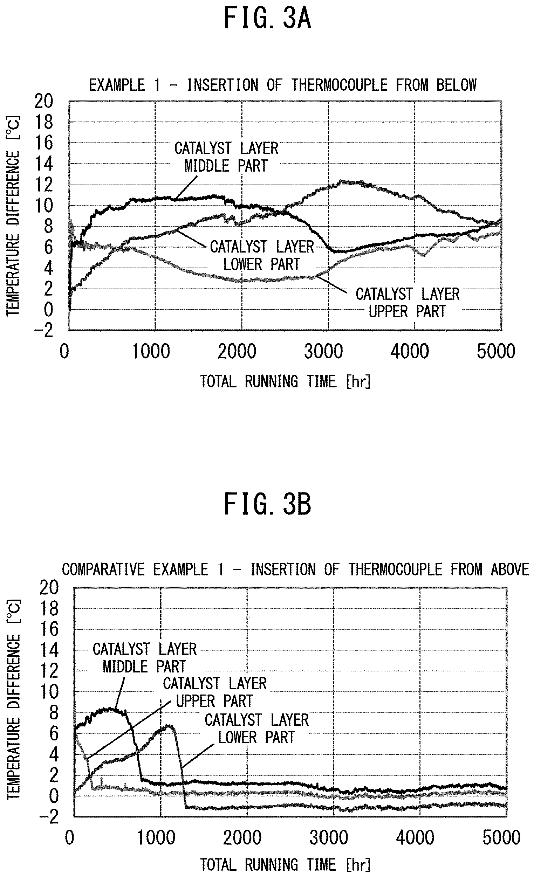

FIGS. 3A and 3B show the temperature differences (catalyst layer temperature - shell temperature) between the catalyst layer temperature and the shell temperature inside the reaction tube 2 in the central part of the reactor 1 during the reaction in Example 1 and Comparative Example 1, respectively. Since the gas-phase catalytic oxidation reaction for synthesizing allyl acetate is an exothermic reaction, when the reaction proceeds normally, the temperature of the catalyst layer becomes higher, i.e., a positive temperature difference is observed.

In Comparative Example 1 (FIG. 3B), no temperature difference was observed after 1000 to 1300 hours from the start of the reaction, though a considerable amount of allyl acetate was produced. This is presumably because the catalyst in the reaction tube 2 having the thermometer protection tube 3 inserted therein was deactivated by the presence of excessive potassium acetate, and the exothermic reaction no longer occurred in the reaction tube, whereby the difference with the shell temperature, which is the temperature outside the reaction tube 2, was eliminated. Since the reaction proceeded normally in reaction tubes 2 having no thermometer protection tube 3 inserted therein, allyl acetate could be produced in the reactor 1 as a whole.

Conversely, in Example 1 (FIG. 3A) in which a thermometer protection tube 3 was inserted into the reaction tube 2 from the bottom part of the reactor 1, a temperature difference was continuously observed even after 1000 hours from the start of the reaction. From this, it can be understood that by inserting a thermometer protection tube from the bottom part of the reactor into the reaction tube, the catalyst layer temperature can be accurately and continuously monitored.

Table 2 shows the amounts of potassium (K) supported on the catalysts of Example 1, Comparative Example 1, and Reference Example 1.

At the start of the reaction, the amount of K supported on the catalyst A was 3.8 mass%, and the amount of K supported on the catalyst B was 2.5 mass%.

Regarding the catalyst after the reaction, in Example 1, the amount of K supported on the catalyst G extracted from the reaction tube 2 having therein a thermometer protection tube 3 inserted from the bottom part of the reactor 1 was 2.9 mass%, and the amount of K supported on the catalyst H was 8.0 mass%. In Comparative Example 1, the amount of K supported on the catalyst E extracted from the reaction tube 2 having therein a thermometer protection tube 3 inserted from the top part of the reactor 1 was 12.6 mass%, and the amount of K supported on the catalyst F was 12.1 mass%, and excess potassium was supported. In Reference Example 1, the amount of K supported on the catalyst C extracted from the reaction tube 2 having no thermometer protection tube 3 was 3.8 mass%, and the amount of K supported on the catalyst D was 8.5 mass%.

From the above results, it can be understood that regarding the K loading amount, the catalyst extracted from a reaction tube 2 having therein a thermometer protection tube 3 inserted from the top part of the reactor 1 behaves very differently from the catalyst in most of the reaction tubes 2 having no thermometer protection tube 3 therein, and thus, is not representative of the catalyst layer as a whole. Conversely, it can be understood that the catalyst extracted from the reaction tube 2 having therein a thermometer protection tube 3 inserted from the bottom part of the reactor 1 behaves similarly to the catalyst in most of the reaction tubes 2 having no thermometer protection tube 3 inserted therein, which indicates that it is representative of the catalyst layer as a whole.

### [Table 2]

**Table 2 - Loading Amount of K on Catalyst (mass%)**

| | Reaction start time (0 h) | | After reaction (7000 h) | |
|---|---|---|---|---|
| Example 1 | A | 3.8 | G | 2.9 |
| | B | 2.5 | H | 8.0 |
| Comparative Example 1 | A | 3.8 | E | 12.6 |
| | B | 2.5 | F | 12.1 |
| Reference Example 1 | A | 3.8 | C | 3.8 |
| | B | 2.5 | D | 8.5 |

### INDUSTRIAL APPLICABILITY

According to the present invention, industrial and stable production of alkenyl acetate is possible.

- 1: fixed-bed multi-tubular reactor
- 2: reaction tube
- 3: thermometer protection tube
- 4: thermometer
- 5: holding device
- 6: jacket
- 7: shell thermometer
- 8: supply piping
- 9: raw material gas supply part
- 10: reaction product discharge part
- 11: extraction piping
- 12: upper affixation plate
- 13: lower affixation plate
- 14: heat medium inlet
- 15: heat medium outlet
- S: raw material gas
- R: reaction product
- SA: alkali metal acetate
- HM: heat medium
- SH: shell

## Claims

1. A fixed-bed multi-tubular reactor for alkenyl acetate production, comprising:
a plurality of reaction tubes which are supplied with a raw material gas and a mist of an aqueous solution of an alkali metal acetate from a top part of the fixed-bed multi-tubular reactor,
a thermometer protection tube which is inserted into at least one of the plurality of reaction tubes from a bottom part of the fixed-bed multi-tubular reactor, and
a thermometer which is inserted into the thermometer protection tube.

2. The fixed-bed multi-tubular reactor according to claim 1, wherein the alkali metal acetate is at least one selected from the group consisting of potassium acetate and cesium acetate.

3. The fixed-bed multi-tubular reactor according to claim 1 or 2, wherein a number of the reaction tubes having the thermometer protection tube inserted therein is 3 to 10.

4. The fixed-bed multi-tubular reactor according to any one of claims 1 to 3, wherein the thermometer is a thermocouple or a resistance thermometer.
